# EUROPEAN PATENT APPLICATION

(11) **EP 4 778 935 A1**
(43) Date of publication of application: **22.07.2026**
(21) Application number: 25151969.0
(22) Date of filing: 15.01.2025
(51) Int. Cl.: C07K 1/16, C07K 1/34, C07K 1/36, B01D 11/04, B01D 61/16

(54) **METHOD FOR PURIFYING A TARGET PRODUCT**

(71) Applicant: Sartorius Stedim Biotech GmbH, 37079 Göttingen (DE)
(72) Inventor: Saballus, Martin, 37079 Göttingen (DE); Kampmann, Markus, 37079 Göttingen (DE)
(74) Representative: Gottschald Patentanwälte Partnerschaft mbB

(57) **Abstract**

The invention relates to a method for purifying a target product, obtained in a bioprocess (1), from an impurity in a process liquid, the method comprising a virus inactivation step (6), a filtration step (7), a first diafiltration step (8) and a first chromatography step (9) as downstream process steps (3). It is proposed that the filtration step (7) is performed after the virus inactivation step (6) and the first diafiltration step (8) is performed after the filtration step (7) and the first chromatography step (9) is performed after the first diafiltration step (8), that the pH value of the process liquid reaches a pH value of smaller than or equal to 4.25 in the downstream process steps (3) before the first diafiltration step (8).

## Description

The present invention relates to a method for purifying a target product according to the general part of claim 1 and to a system for purifying a target product according to the general part of claim 15.

In bioprocessing, the purification of a target product from one or more impurities in a given process liquid is a cost- and labor-intensive process. For example, for some target products the purification may account for up to 85% of the total costs of the final product. Consumables, such as expensive chromatography media, in particular Protein A chromatography media, and long process times are factors accounting for a majority of the purification costs. In many cases, the purification consists of many individual downstream process steps which all contribute to the overall process time and costs. In order to reduce these costs, measures such as continuous downstream steps, e.g. continuous chromatography, have been developed to reduce material consumption and to speed up process times.

The known prior art (WO 2021/089770 A2) that builds the basis of the invention is related to a method for purifying a protein as a target product, obtained in a bioprocess, from an impurity in a process liquid. This method comprises an adjustment of the pH of the process liquid to precipitate host cell proteins (HCPs) in the process liquid. Following the HCP precipitation the method comprises a filtering of the process liquid through a filter material, namely a depth filter material, where at least a part of the impurity in form of precipitated HCP is separated from the target product remaining in the process liquid by the filter material. In a first diafiltration step, which is following the depth filter step, the process liquid is filtered through a diafiltration membrane. This step is then followed by a first chromatography step which comprises a contacting of the process liquid with a first chromatography medium. In this first chromatography step, at least a part of the impurity is separated from the target product by the first chromatography medium. The first chromatography step is followed by a virus inactivation step which comprises an adjustment of the pH to a value of 3.0 - 5.0 which is suitable for inactivating a virus in the process liquid.

The known method results in undesirable long process times and high production costs for the final target product.

It is therefore a challenge to provide a cost-efficient method for purifying a target product in a bioprocess.

The invention is based on the problem of providing such a method.

The above-noted object is solved by the features of the characterizing part of claim 1.

The main realization of the present invention is, that by performing the virus inactivation step prior to the filtration step, it becomes possible to combine the virus inactivation step with following downstream process steps. A virus inactivation step as an independent step can therefore be omitted or shortened which results in shorter process times and therefore reduced production costs for the final target product or can at least be integrated into the downstream process steps more efficiently.

As a pH value of smaller than or equal to 4.25 is known to sufficiently inactivate a viral contamination of the process liquid, the inactivation of viral contamination can, for example, be achieved by adjusting the pH value of the process liquid to a pH value of smaller than or equal to 4.25 in the downstream process steps before the filtration step and thus in an early downstream process step. As a pH value of smaller than or equal to 4.25 is further known to induce the precipitation of an impurity, in particular of HCPs, polynucleotides and cell debris, hereinafter denoted as production system based impurities, such low pH values enable the precipitation of at least a part of the impurity within the virus inactivation step. The precipitation of impurities facilitates their removal in the subsequent filtration step, which reduces the risk of fouling of materials used in the downstream process steps following the filtration step. This may ultimately result in a reduced process time and reduced production costs for the target product.

As the precipitation is known to be a fast process compared to the required time for virus inactivation, the virus inactivation step can be combined with the following filtration step which allows the removal of at least a part of the impurity, in particular a precipitated production system based impurity, while simultaneously inactivating a virus in the process liquid. Combining the virus inactivation and filtration can therefore lead to drastically reduced process times and production costs for the final product.

Advantageously, the first diafiltration step can be used to readjust the pH value to a higher value, e.g. higher than 4.25. By providing sufficient incubation time between the precipitation and the diafiltration, the virus inactivation may be finished. The higher pH value may lead to a dissolution of at least a part of the impurity, in particular a production system based impurity, which may be advantageous for the following downstream process steps, e.g. by increasing the life-time of the first chromatography medium of the first chromatography step and/or by increasing the filter capacity of following filtration steps. Adjusting the pH value of the process liquid to a pH value of higher or equal to 4.25 after the first diafiltration step may also provide a more stable and milder environment of the target product. The pH value of 4.25 offers a good boundary for an efficient viral inactivitation. Lower pH values, for example at most 3.6, are also preferred, in particular with regard to regulatory demands.

In detail, it is proposed that the filtration step is performed after the virus inactivation step and the first diafiltration step is performed after the filtration step and the first chromatography step is performed after the first diafiltration step, that the pH value of the process liquid reaches a pH value of smaller than or equal to 4.25 in the downstream process steps before the first diafiltration step.

In a preferred embodiment according to claim 2, the target product is an antibody or a monoclonal antibody.

As every downstream process step of the downstream process steps which involves the use of Protein A is expensive, the overall production costs can be reduced if all of the downstream process steps are free of Protein A (claim 3).

Claim 4 specifies the first and last downstream process step of the downstream process steps to be a harvest step and a final product collection step, respectively.

Claim 5 specifies the parameter of the process liquid to be the temperature and/or the concentration of a virus inactivating agent such as a pH altering substance and/or a solvent and/or a detergent. Adjusting the temperature and/or altering the pH value by addition of the pH altering substance allows for an effective virus inactivation for specific types of viruses, as some viruses are sensitive to certain temperature and pH ranges. Employing a solvent can effectively denature or disrupt a virus, thus rendering it inactive, whereas a detergent can help break down the lipid envelope of certain viruses, ultimately leading to their inactivation.

According to embodiments of claim 6, the pH value of the process liquid remains constant between the virus inactivation step and the first diafiltration step. Keeping the pH value equal to or below 4.25 between the virus inactivation step and the first diafiltration step allows for combining the virus inactivation step with all downstream process steps between the virus inactivation step and the ultrafiltration step, such as the precipitation step, the filtration step and a possible storage step of the process liquid, ultimately leading to drastically reduced process times and production costs.

Claim 7 specifies an adjustment of the pH value of the process liquid to an inactivation pH value of smaller than or equal to 4.25 in the virus inactivation step by means of the addition of a pH altering substance to the process liquid.

According to embodiments of claim 8, the first diafiltration step involves an adjustment of the pH value and/or the conductivity of the process liquid. An adjustment of these parameters allows stabilization of the product and providing optimized conditions for the purification of the target product in the following downstream process steps, especially when the properties, in particular the physicochemical properties which are decisive for the purification of the target product in the corresponding step, are also taken into consideration. An adjustment of the pH value of the process liquid to a pH value which is higher than an inactivation pH may result in a high binding affinity of the target product as well as a higher selectivity against impurities to the first chromatography medium thereby leading to a low target product loss. Alternatively or additionally, adjusting the conductivity value of the process liquid to a value of below 25 mS/cm may positively affect the binding affinity of the target product to the first chromatography medium, which may also lead to a reduced target product loss as well as high binding capacities and thus high resin utilization.

According to preferred embodiments of claim 9, the first chromatography step may be the first chromatography step of the downstream process steps. In other embodiments according to claim 9, the first chromatography step may alternatively or additionally directly be followed by a group of interrelated further chromatography steps. This group of further chromatography steps comprises at least a second chromatography step which involves a contacting of the process liquid with a second chromatography medium. Providing a group of interrelated further chromatography steps allows for the purification of the target protein using different chromatography media, ultimately resulting in a high target product purity.

Claim 10 specifies the first and second chromatography medium. In preferred embodiments according to claim 10, the first and the second chromatography medium are immunoglobulin binding protein free which can result in low production costs as expensive immunoglobulin binding protein is omitted.

According to embodiments of claim 11, the downstream process steps comprise a centrifugal clarification step where at least a part of the impurity, in particular a particulate impurity, is separated from the target product by a centrifuge. Employing a centrifuge may result in a fast and efficient removal of at least a part of the impurity, in particular of a particulate impurity such as cells and cell debris. In preferred embodiments, the centrifugal clarification step is performed before the virus inactivation step which allows for a removal of cells and other particulate material in an early downstream process step. Such preferred embodiments may further allow a fast processing of the process liquid and reduce the required filter area in following filtration steps. Such preferred embodiments can also prevent a release of one or more impurities from the cells in further downstream process steps which may reduce the amount of the impurity to be separated from the target product in the further downstream process steps such as the number of precipitated particles to be separated in the following filtration step.

In other preferred embodiments, the centrifuge is a continuous centrifuge, in particular a fluidized bed centrifuge. Such embodiments may increase the productivity of the overall purification process and thus reduce the production costs for the target product.

In embodiments according to claim 12, the downstream process steps further comprise an ultrafiltration step, which involves the filtering of the process liquid through an ultrafiltration membrane. Such embodiments allow for an increase in the concentration of the target product in the process liquid and for a reduction of the process liquid volume and may further compensate for dilutions of the target product which may be caused by a washing step of a preceding centrifugation step when a continuous centrifuge is used and/or by the use of a pH altering substance in the virus inactivation step. Increasing the target product concentration and reducing the process liquid volume can ultimately lead to a faster and more efficient purification of the target product. In preferred embodiments the ultrafiltration step is performed before the first diafiltration step which may result in a volume reduction in a very early downstream process step, thereby facilitating the following downstream process steps, in particular following chromatography steps. Furthermore, due to the increase in concentration in the ultrafiltration step, the volume of a diafiltration buffer required in the diafiltration step can be saved which may also contribute to lower production costs for the target product.

In embodiments according to claim 13, the downstream process steps further comprise a flocculation step which involves an addition of a flocculation inducing agent to the process liquid. In such embodiments, finest suspended or colloidal solid particles (smaller than 1 µm) such as small particles of a precipitated production system based impurity, can be aggregated to form larger particles which allows for fast and efficient removal of those larger particles by sedimentation or by filtration. This may simplify following downstream process steps and increase the target product purity.

According to preferred embodiments of claim 14, the ultrafiltration step and the first diafiltration step are performed in a single-pass-mode, in which the process liquid passes the ultrafiltration and diafiltration membrane only once. This mode of operation allows a continuous ultrafiltration/diafiltration of the process liquid which results in high productivity and short process times.

Another teaching according to claim 15, which is of equal importance, relates to a system for purifying a target product.

Here, it is essential that the filtration step is performed after the virus inactivation step and the first diafiltration step is performed after the filtration step and the first chromatography step is performed after the first diafiltration step, that the pH value of the process liquid reaches a pH value of smaller than or equal to 4.25 in the downstream process steps before the first diafiltration step.

All explanations given with regard to the proposed method are fully applicable.

In the following, embodiments of the invention are explained with respect to the drawing. The drawing shows in
- Fig. 1: a schematic representation of a proposed method,
- Fig. 2: the total yield of a mAb as target product and the yield of the mAb in each downstream process step of an embodiment of the proposed method,
- Fig. 3: the content and reduction of DNA as a first impurity in each downstream process step of the proposed method according to Fig. 2 and
- Fig. 4: the content and reduction of HCP as a second impurity in each downstream process step of the proposed method according to Fig. 2 and Fig. 3.

Proposed is a method for purifying a target product, obtained in a bioprocess 1, from an impurity in a process liquid. A bioprocess 1 is a single or a series of biological and/or chemical and/or physical operations used to produce a target product in a target product formation process. The bioprocess 1 may comprise an upstream process step which may involve the cultivation of living cells and/or enzymes to convert raw materials, for example, nutrients in a cultivation medium, into the target product through a controlled process. Preferably, the cultivation of cells is performed in a bioreactor 2. The living cells may be selected from the group of prokaryotic cells such as *Escherichia coli* or from the group of eukaryotic cells such as mammalian cells. In some embodiments, the mammalian cells can be Chinese hamster ovary (CHO) cells, HEK293 cells, mouse myeloma (NS0) cells, baby hamster kidney (BHK) cells, monkey kidney fibroblast (COS-7) cells, Madin-Darby bovine kidney (MDBK) cells or any combination thereof. The target product may for example be an antibody.

An impurity is any substance which differs from the target product in its desired physical and/or chemical parameters. The impurity may be soluble or insoluble in the process liquid and may, for example, be distributed in the process liquid in the form of solid particles. Non-limiting examples for the impurity are cells, cell debris, host cell proteins (HCP), nucleic acids such as DNA or RNA, and/or viral contaminants.

The term "purifying a target product from an impurity" refers to the separation of multiple different species of impurities from the target product and/or to the separation of a part of an impurity of a single species from the target product.

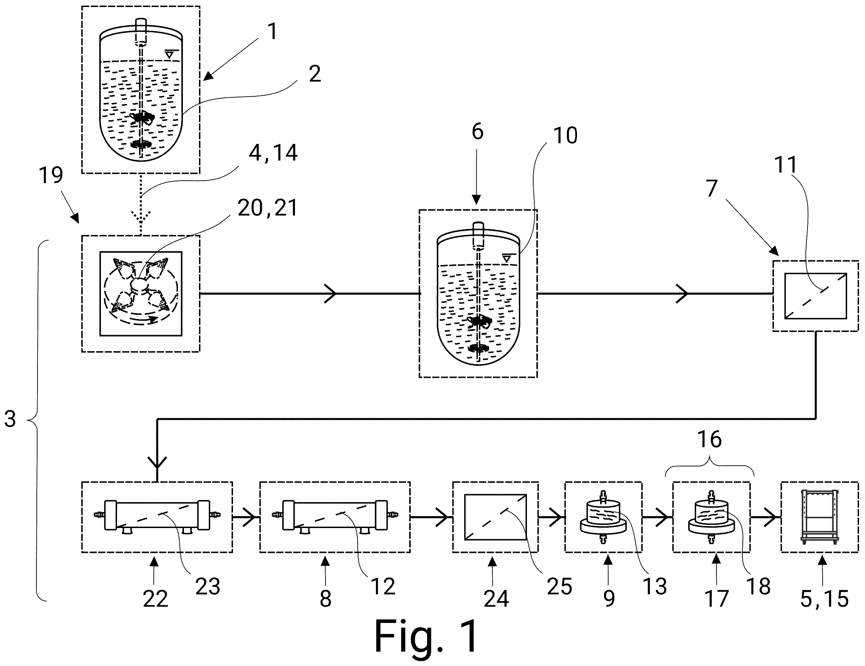

The proposed method as schematically shown in Fig. 1 comprises purifying the target product in several downstream process steps 3. A downstream process step 3 is any process step that occurs after the target product formation process. For example a downstream process step 3 may be used to store, recover, purify, concentrate, stabilize and/or formulate the target product or to increase or ensure the pharmaceutical safety of the target product. The process liquid is transferred from a first downstream process step 4 of the downstream process steps 3 to a last downstream process step 5 of the downstream process steps 3, preferably by at least one transport mechanism (not shown). The transport mechanism may comprise a pump, preferably the transport mechanism comprises a peristaltic pump.

A process liquid is any liquid obtained in the bioprocess 1 that comprises at least one target product and at least one impurity and that is subjected to the several downstream process steps 3 for purifying the target product. The process liquid may comprise a further component such as a buffer component. In an exemplary embodiment, the process liquid is a cell culture broth comprising CHO cells as an impurity and an antibody as the target product.

As shown in Fig. 1, the downstream process steps 3 comprise a virus inactivation step 6, a filtration step 7, a first diafiltration step 8 and a first chromatography step 9.

The virus inactivation step 6 comprises an adjustment of at least one parameter of the process liquid to a value which is suitable for inactivating a virus. The term "inactivating a virus" means rendering a virus unable to replicate or to multiply which can be achieved by disrupting the virus's ability to infect cells or by inhibiting the virus ability to reproduce or to spread. The virus inactivation step 6 may be an independent downstream process step 3 in which only the virus inactivation takes place, or it may be a downstream process step 3 which is combined with at least one following downstream process step 3 such as the filtration step 7 and/or a precipitation step. As shown in Fig. 1, the virus inactivation may be performed in a single tank 10, preferably a stirred tank 10. In some embodiments, the process liquid may - after adjustment of the at least one parameter of the process liquid - be transferred to the at least one following downstream process step 3, in order to combine the virus inactivation step 6 with the at least one following downstream process step 3. In other embodiments, the adjustment of the at least one parameter of the process liquid may be performed using a continuous mixing while the process liquid flows (not shown) instead of a tank 10. The at least one parameter of the process liquid may be adjusted to a certain value for a predefined time, hereinafter referred to as "incubation time" which is the time after the final adjustment of the at least one parameter of the process liquid until an intended change of the at least one parameter of the process liquid. Preferably, the incubation time is between 15 seconds and 120 minutes, more preferably between 30 minutes and 90 minutes, more preferably between 15 minutes and 70 minutes and 60 min.

The filtration step 7 comprises a filtering of the process liquid through a filter material 11, where at least a part of the impurity, in particular a particulate impurity, is separated from the target product by the filter material 11. The term "filter material" is to be understood wide and refers to any physical barrier that allows a portion of the process liquid to pass through while trapping and retaining at least a part of the impurity in dependency of the size of the impurity on its surface and/or within its interior. Preferably, the filtration step 7 is a bag filter step and/or a depth filter step. The filtration step 7 may involve the addition of a filter aid to the process liquid. The filter aid may be diatomaceous earth. Preferably, the pH value of the process liquid is smaller than or equal to 4.25 in the filtration step 7. A pH value of smaller than or equal to 4.25 may result in an adsorption of at least a part of the impurity to the filter material 11 and/or to the filter aid which results in a very high purity of the target product already after the filtration step 7.

The filtration step 7 can be performed either in normal flow filtration mode or in tangential flow filtration mode. Normal flow filtration is a type of filtration process where the process fluid flows perpendicular to the filter material 11, and the particles or contaminants are retained on the surface of the filter rather than passing through it. A filtration performed in normal flow filtration mode is inexpensive and easy to scale up. In tangential flow filtration mode the process fluid is directed tangentially across the filter material 11 surface rather than perpendicular to it..

As shown in Fig. 1, the first diafiltration step 8 comprises a filtering of the process liquid through a first diafiltration membrane 12. Preferably, in the first diafiltration step 8, a buffer component of the process liquid is exchanged by another buffer component, therefore resulting in a buffer exchange of the process liquid. It is preferred that the first diafiltration step 8 results in a change of the pH value of the process liquid, preferably in an increase of the pH of the process liquid. The diafiltration membrane may have a molecular weight cutoff (MWCO) between 3 and 1000 kDa, preferably between 5 and 100 kDa more preferably between 10 kDa and 30 kDa. This range is particularly relevant for the purification of antibodies.The diafiltration membrane material may comprise polyethersulfone, polysulfone, regenerated cellulose and/or cellulose triacetate.

The first chromatography step 9 comprises a contacting of the process liquid with a first chromatography medium 13, where at least a part of the impurity is separated from the target product by the first chromatography medium 13. The target product is separated from at least a part of the impurity as a result of differences in rates at which the target product and the part of the impurity which is separated in the chromatography step migrate through the chromatography medium under the influence of a moving process liquid and/or in differences in the adsorption behavior of the target product and the part of the impurity which is separated in the chromatography step by the chromatography medium. The chromatography medium may comprise a ligand which interacts with the target product and/or at least a part of the impurity.

The first chromatography step 9 may be performed in a flow-through mode or in a capture mode, preferably in a capture mode.

It is essential that the filtration step 7 is performed after the virus inactivation step 6 and the first diafiltration step 8 is performed after the filtration step 7 and the first chromatography step 9 is performed after the first diafiltration step 8. It is further essential that the pH value of the process liquid reaches a pH value of smaller than or equal to 4.25 in the downstream process steps 3 before the first diafiltration step 8. It is possible additionally, that the pH value of the process liquid remains higher or equal to 4.25 after the first diafiltration step 8.

The target product may be selected from the group of viruses, virus-like particles, small molecules or proteins. The target product may be a therapeutic protein. In some aspects, the target product is a Fc fusion protein, an anticoagulant, a blood clotting factor, an engineered protein scaffold, an enzyme, a growth factor, a hormone, an interferon, an interleukin, a receptor, or a thrombolytic. In some aspects, the target product is a recombinant protein.

In particularly preferred embodiments, the target product is an immunoglobulin, hereinafter referred to as antibody. The antibody may be a human antibody, a humanized antibody, or a chimeric antibody. In certain embodiments, the target product is a bispecific antibody. A "bispecific" or "bifunctional antibody" is an artificial hybrid antibody having two different heavy/light chain pairs, giving rise to two antigen binding sites with specificity for different antigens.

In other embodiments the target product may be a chimeric polypeptide comprising an antigen binding fragment of an antibody. In other embodiments the target product is a viral vector such as an adeno-associated virus or a lentivirus. Preferably, the target product is a monoclonal antibody ("mAb").

Here and preferably, all of the downstream process steps 3 are Protein A free. Although Protein A allows - due to its specific binding to antibodies - to concentrate an antibody as target product while simultaneously drastically increasing the purity of the antibody, the production of Protein A and therefore its use in the downstream process steps 3 is expensive. As a process liquid that reaches a pH value of smaller than or equal to 4.25 in the downstream process steps 3 before the first diafiltration step 8 may result in precipitation of at least a part of the impurity, which can then be removed in the filtration step 7, the purity of the target product can drastically be increased, thereby allowing to omit the expensive use of Protein A.

According to one embodiment it is proposed that the first downstream process step 4 of the downstream process steps 3 is a harvest step 14 and that the last downstream process step 5 of the downstream process steps 3 is a final product collection step 15. Preferably, the harvest step 14 involves the harvest of mammalian cells, in particular CHO cells.

Here and preferably, the at least one parameter of the process liquid to be adjusted in the virus inactivation step 6 is the temperature of the process liquid and/or the concentration of a virus inactivating agent in the process liquid. The term "virus inactivating agent" refers to any substance that inactivates a virus present in the process liquid. The virus inactivating agent may be added to the process liquid, preferably in a predefined concentration.

Preferably, the virus inactivating agent is a pH altering substance and/or a solvent and/or a detergent. The pH altering substance may be an acid such as lactic acid, ascorbic acid, acetic acid, hydrochloric acid, phosphoric acid, citric acid, glycine, succinic acid and/or sulfuric acid. Preferably, the altering substance may comprise an acid with a titratable group with a pKs between 2.0 and 4.75. The virus-inactivating conditions in the process liquid may be selected so that the concentration of the acid in the process liquid can be up to 200 mM and still have sufficient buffer properties to enable effective virus inactivation without damaging the target product, e.g. by acidic denaturation.

In some embodiments, the pH value of the process liquid remains constant between the virus inactivation step 6 and the first diafiltration step 8. The term "between the virus inactivation step 6 and the first diafiltration step" refers to the time from the moment when the desired pH value is reached in the virus inactivation step 6 until the beginning of the diafiltration step, preferably to the moment when an intended change of the pH occurs during the diafiltration step. The term "constant" refers to a pH fluctuation of ± 0.1 pH units. Preferably, no pH altering substance is added to the process liquid between the virus inactivation step 6 and the first diafiltration step 8. Such embodiments allow an efficient precipitation and removal of a production system based impurity such as DNA resulting in reduced process times and thus production costs of the target product.

According to one embodiment it is proposed, that the pH value of the process liquid in the downstream process steps 3 before the first diafiltration step 8 reaches a pH value of above 3.1 and/or below 3.6, preferably, 3.4.

Preferably, the pH value of the process liquid is adjusted in the virus inactivation step 6 to an inactivation pH value of smaller than or equal to 4.25, in particular to a pH value of above 3.1 and/or below 3.6, more preferably, 3.4, by addition of the pH altering substance to the process liquid. Preferably, the incubation time is 60 min at a pH value of 3.4 as this condition may result in a virus inactivation which meets the demands of the regulatory authorities.

According to one embodiment it is proposed, that the first diafiltration step 8 involves an adjustment of the pH value of the process liquid. This adjustment may be achieved by separating a part of the process liquid through the first diafiltration membrane 12 (permeate) while keeping the target product in the portion of the process liquid which is retained by the first diafiltration membrane 12 (retentate) and simultaneous or subsequent addition of a pH adjusting liquid with the desired pH to the retentate which compensates for the volume loss.

Preferably, the pH value of the process liquid is adjusted in the first diafiltration step 8 to a pH value which is higher than the inactivation pH value of the virus inactivation step 6, more preferably, that the pH value of the process liquid is adjusted in the first diafiltration step 8 to a pH value of higher or equal to 4.25, preferably higher or equal to 4.5, more preferably higher or equal to 5.0, most preferably higher or equal to 5.5. An adjustment of the pH value of the process liquid which is higher than the inactivation pH of the virus inactivation step 6 may advantageously result in a termination of the virus inactivation and a stabilization of the target product. A pH value of higher or equal to 4.25 may further result in a dissolution of a particulate part of the impurity which may increase the life-time of the first chromatography medium 13, e.g. by reduced fouling of the first chromatography medium 13. Furthermore, a pH value of higher or equal to 4.25, in particular a pH value of 5.5 or higher may be optimal for a purification of the target product in the first chromatography step 9 and may in particular result in a high binding affinity of the target product to the first chromatography medium 13 and/or in a high selectivity against impurities.

Alternatively or additionally, the first diafiltration step 8 involves an adjustment of the conductivity value of the process liquid. This adjustment may be achieved by separating a part of the process liquid through the first diafiltration membrane 12 while keeping the target product in the retentate and simultaneously or subsequently adding a liquid with the desired conductivity to the retentate which compensates for the volume loss.

Preferably, the conductivity value of the process liquid is adjusted in the first diafiltration step 8 to a pH value of lower or equal to 25.0 mS/cm, preferably lower or equal to 20.0 mS/cm, more preferably lower or equal to 15.0 mS/cm, more preferably lower or equal to 10.0 mS/cm, most preferably lower or equal to 5.0 mS/cm. A conductivity of 25 mS/cm and below may provide a high binding affinity and a high binding capacity of the target product to an ion exchange chromatography medium.

According to one embodiment it is proposed, that the first chromatography step 9 is the first chromatography step 9 of the downstream process steps 3. Additionally or alternatively, the first chromatography step 9 is directly followed by a group of interrelated further chromatography steps 16, wherein the group of interrelated further chromatography steps 16 comprises at least a second chromatography step 17 (Fig. 1), wherein the second chromatography step 17 comprises a contacting of the process liquid with a second chromatography medium 18. In the second chromatography step 17 at least a part of the impurity is separated from the target product by the second chromatography medium 18. Preferably, the last chromatography step of the group of interrelated further chromatography steps 16 is the last chromatography step of the downstream process steps 3 (Fig. 1). Each chromatography step of the group of interrelated further chromatography steps 16 may be performed in capture mode or flow-through mode. It is particularly preferred, that the first chromatography step 9 is performed in capture mode and that the second chromatography step 17 is performed in flow-through mode.

According to one embodiment it is proposed, that the first chromatography medium 13 and/or the second chromatography medium 18 is a cation exchange chromatography medium, an anion exchange chromatography medium, a hydrophobic chromatography medium, a mixed-mode chromatography medium or an affinity chromatography medium. Preferably, the first chromatography medium 13 is a cation exchange chromatography medium and the second chromatography medium 18 is an anion exchange chromatography medium which allows an efficient removal of at least a part of the impurity by employing two orthogonal chromatography steps, which may result in a high purity of the target product. In some embodiments the first chromatography medium 13 and/or the second chromatography medium 18 may be an affinity chromatography medium which specifically binds host cell proteins of mammalian cells. Preferably, the first chromatography medium 13 is a particulate chromatography medium and the second chromatography medium 18 is a membrane.

It is preferred, that the first chromatography medium 13 and the second chromatography medium 18 are immunoglobulin binding protein free, in particular Protein A free, which may result in reduced production costs for the target product as the production costs for immunoglobulin binding proteins and thus its use is expensive. Thus, it is particularly preferred, that any one of the chromatography steps of the downstream process steps 3 is immunoglobulin binding protein free, in particular Protein A free.

As shown in Fig. 1, the downstream process steps 3 may further comprise a centrifugal clarification step 19, where at least a part of the impurity is separated from the target product by a centrifuge 20. Preferably, the centrifuge 20 is a continuous centrifuge 21, in particular a fluidized bed centrifuge. As also shown in Fig. 1 it is in particular preferred, that the centrifugal clarification step 19 is performed before the virus inactivation step 6. In the centrifugal clarification step 19 particles, such as cells, can be removed in an early downstream process step 3, which may reduce the number of particles prior to the filtration step 7 ultimately resulting in a higher filter capacity of the filter material 11. Furthermore, the removal of cells in an early downstream process step 3 may prevent a release of cytoplasm caused by a rupture of cells in the virus inactivation step 6 as a result of a decrease in pH. A release of cytoplasm however may result in a release of intracellular impurities which complicates the purification of the target product.

According to one embodiment it is proposed, that the downstream process steps 3 further comprise an ultrafiltration step 22, wherein the ultrafiltration step 22 comprises a filtering of the process liquid through an ultrafiltration membrane 23, wherein the ultrafiltration step 22 comprises an increase in the concentration of the target product in the process liquid. By increasing the concentration of the target product, the volume of the process liquid is reduced which simplifies and accelerates the subsequent downstream process steps 3 which may ultimately lead to reduced target product cost. As shown in Fig. 1 it is preferred, that the ultrafiltration step 22 is performed before the first diafiltration step 8, in particular between the filtration step 7 and the first diafiltration step 8.

Preferably, and as shown in Fig. 1, the downstream process steps 3 may further comprise a sterile filtration step 24 which comprises a filtering of the process liquid through a sterile-filtration membrane 25. Preferably the sterile filtration membrane 25 has a nominal retention rating of smaller than or equal to 0.2 µm. In the sterile filtration step 24, at least a part of the impurity, in particular a particulate impurity, is separated from the target product by the sterile-filtration membrane 25. The sterile filtration may be performed prior to the first chromatography step 9 which may increase the life-time of the first chromatography medium 13. Alternatively or additionally, the or another sterile filtration step 24 is performed after the last chromatography step of the downstream process steps 3 in order to provide a pathogen free process liquid after the last chromatography step. Alternatively or additionally, the downstream process steps 3 may further comprise a virus filtration step which comprises a filtering of the process liquid through a virus-filtration material (not shown). It is preferred that the virus filtration step is performed after the last chromatography step of the downstream process steps 3. In other embodiments, the optional sterile filtration membrane 25 and/or the filter material 11 may additionally comprise a fleece material, in particular a glass fiber fleece material, in particular to increase the filter capacity of the sterile filtration step.

In preferred embodiments the first diafiltration membrane 12 and the ultrafiltration membrane 23 are arranged together in one housing. In another embodiment the first diafiltration membrane 12 and the ultrafiltration membrane 23 and the sterile-filtration membrane 25 are arranged together in one housing.

According to one embodiment it is proposed, that the downstream process steps 3 further comprise a flocculation step, wherein the flocculation step comprises an addition of a flocculation inducing agent to the process liquid, where at least a part of the impurity is flocculated by the flocculation inducing agent. The flocculation provides an aggregation of small particulate impurities to larger particles which can be easily removed by filtration. It is therefore preferred that the flocculation step is performed directly before the filtration step 7 which may result in a drastically increased purity of the target product ultimately after the filtration step 7. The flocculation inducting agent may be polydi-allyldimethylammonium chloride, preferably in a concentration of 0.01 % (w/v) to 0.2 % (w/v).

It is preferred, that the flocculation step and the virus inactivation step 6 are a combined step, or, that the flocculation step is performed directly after the virus inactivation step 6.

In some embodiments the downstream process steps 3 further comprise a second diafiltration step, wherein the second diafiltration step comprises a filtering of the process liquid through a second diafiltration membrane. Preferably, the second diafiltration step is performed after the first chromatography step 9.

The ultrafiltration membrane 23 and/or the first diafiltration membrane 12 and/or the second diafiltration membrane may be a flat sheet membrane. In preferred embodiments the ultrafiltration membrane 23 and/or the first diafiltration membrane 12 and/or the second diafiltration membrane is a hollow fiber membrane. A hollow fiber ultrafiltration membrane 23 is less affected by particles larger than 0.2 µm which allows omitting a preceding sterile filtration step 24. Due to a high robustness of hollow fiber membranes against particles, possible precipitations that might occur in the ultrafiltration step 22 and/or in the first diafiltration step 8 do not impair these downstream process steps 3. One of the cost drivers of downstream processing is an extensive use of sterile filters. Here and preferably, the downstream process steps 3 comprise only sterile filtration steps 24 after the first diafiltration step and preferably only a single sterile filtration step 24. That may be the case due to the hollow fiber membranes even though submicrometer cell fragments are usually not removed by a fluidized bed centrifuge. A hollow fiber membrane may also reduce stress on sensitive molecules and cells. Using two separate membranes for the ultrafiltration and the diafiltration in a single pass embodiment allows efficiently dealing with precipitations while at the same time keeping the process time low.

According to some embodiments it is proposed, that the ultrafiltration step 22 and the first diafiltration step 8 are performed in a single-pass-mode, in which the process liquid passes the ultrafiltration membrane 23 and the first diafiltration membrane 12 only once, that the ultrafiltration step 22 is followed by the first diafiltration step 8, in particular directly followed by the first diafiltration step 8, or, that the first diafiltration step 8 is followed by the ultrafiltration step 22, in particular directly followed by the ultrafiltration step 22. Preferably, the ultrafiltration membrane 23 and the first diafiltration membrane 12 are continuously flown through by the process liquid. Such an embodiment allows a fast and efficient combined ultrafiltration and diafiltration. Preferably, the ultrafiltration step 22 and the first diafiltration step 8 are performed in tangential flow filtration mode. In preferred embodiments the ultrafiltration membrane 23 and the first diafiltration membrane 12 are housed in one module.

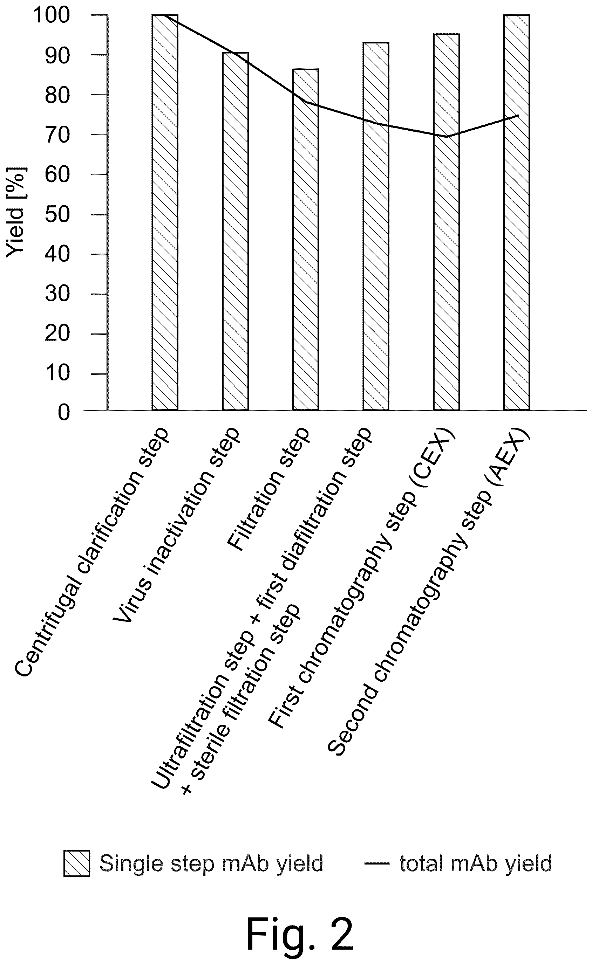

A preferred embodiment of the method for purifying a mAb as target product, obtained in a bioprocess 1 in form of a cell culture, comprises the following downstream profess steps in the following order: a harvest step 14 as the first downstream process step 4 of the downstream process steps 3, a centrifugal clarification step 19, a virus inactivation step 6, a depth filter step as filtration step 7, an ultrafiltration step 22, a first diafiltration step 8, a sterile filtration step 24, a first chromatography step 9 and a second chromatography step 17. In the centrifugal clarification step 19 at least a part of the impurity is separated from the target product by a fluidized bed centrifuge 20. The subsequently following virus inactivation step 6 involves an adjustment of the pH value of the process liquid to a pH of 3.4 and a subsequent storage of the process liquid for 30 minutes in a tank 10. In the ultrafiltration step 22, the target product is concentrated by a factor of 5 to 10. An adjustment of the pH value of the process liquid to a pH from 3.4 to 5.5 and a reduction of the conductivity of the process liquid to less than 5 mS/cm is then performed in the first diafiltration step 8. The conditions in the filtration step 7 and the ultrafiltration step 22 are set so that both steps together take about 30 minutes. Together with the storage of the process liquid for 30 minutes in the tank 10, a total incubation time for virus inactivation of about 60 minutes of the process liquid at a pH of 3.4 is achieved until the pH of the process liquid is adjusted to 5.5 in the first diafiltration step 8. The pH value of 3.4 of the process liquid thus remains constant between the virus inactivation step 6 and the first diafiltration step 8 for 60 minutes. The first chromatography step 9 comprises a contacting of the process liquid with a particulate cation exchange chromatography medium as first chromatography medium 13. The second chromatography step 17 comprises a contacting of the process liquid with a membrane anion exchange chromatography medium as the second chromatography medium 18. The first chromatography step 9 is performed in capture mode and the second chromatography step 17 is performed in flow-through mode. Results of this preferred embodiment are shown in figures 2 to 4.

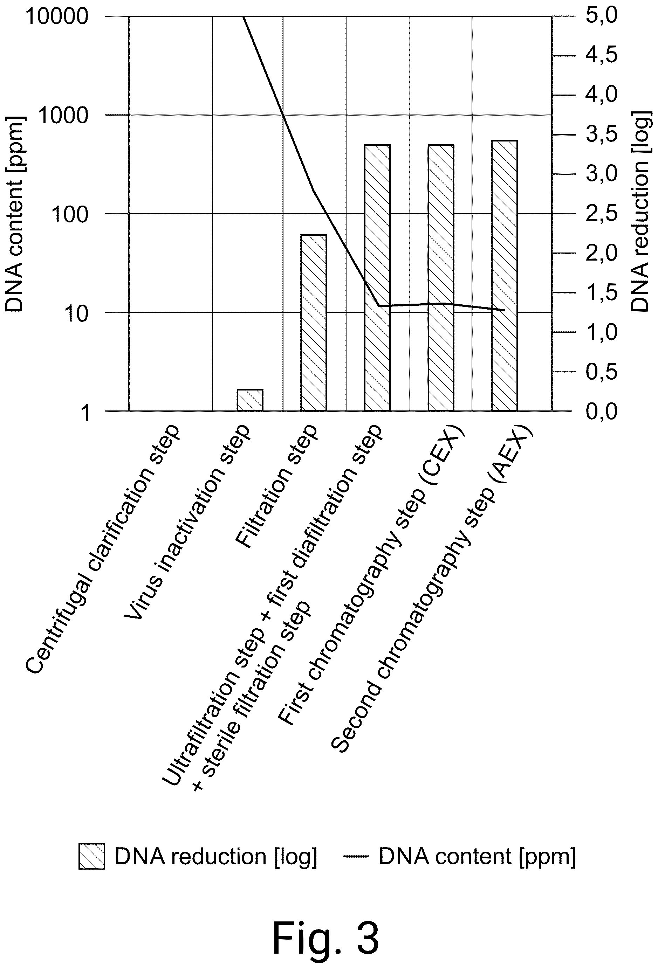

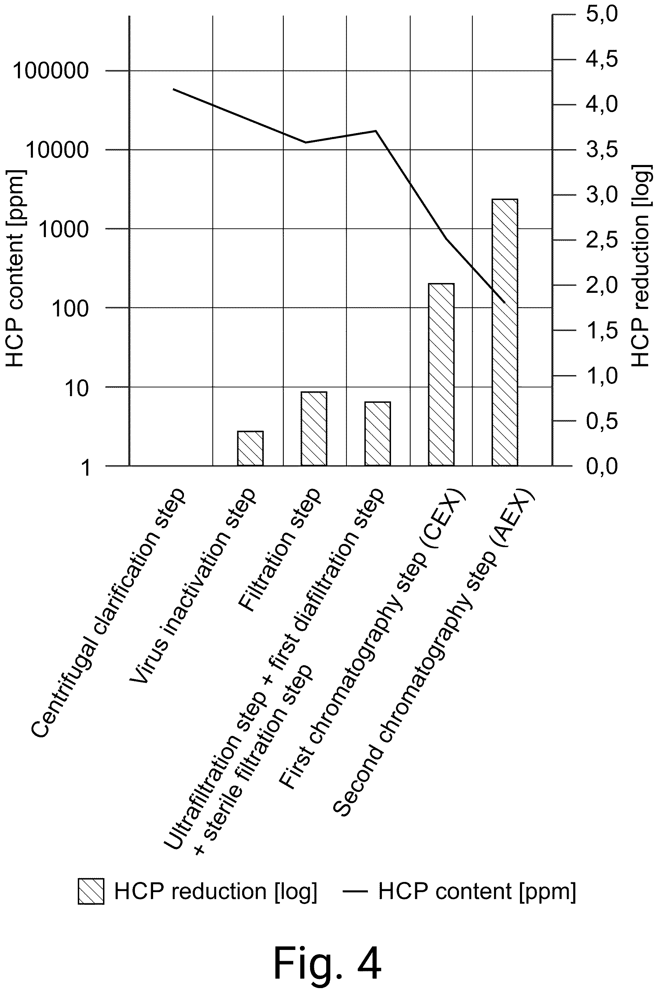

Fig. 2 shows that a high total yield of around 70 % of a mAb after the second chromatography step 17 can be achieved by the proposed method. As shown in Fig. 3, the content of DNA as a first part of the impurity is reduced from 17601 ppm after the centrifugal clarification step 19 to 10 ppm after the sterile filtration step 24 (Fig. 3) which corresponds to a reduction of more than 99.9 % of the DNA content. As shown in Fig. 3, the DNA content remains almost constant after the sterile filtration step 24 resulting in a final DNA content of 9 ppm after the second chromatography step 17. Fig. 3 thus shows that the majority of DNA can be removed by performing the virus inactivation step 6 before the filtration step 7, which is the result of precipitated DNA at the low pH of 3.4 which is efficiently be removed in the subsequent filtration steps. Fig. 4 shows that the content of HCP as a second impurity is reduced from 60599 ppm after the centrifugal clarification step 19 to 78 ppm after the second chromatography step 17, which corresponds to a reduction of more than 99.8 %. The final HCP content is well below 100 ppm which is typically regarded as an upper limit by the regulatory authorities.

The final yield of the mAb as target product and also the reduction of the DNA and HCP as first and second part of the impurity are comparable to other methods that comprise at least one downstream process step 3 involving the use of immunoglobulin binding protein such as Protein A. Thus, the data presented in figures 2-4 verify how the proposed method can ultimately lead to a substantial reduction of the impurity without the use of any expensive immunoglobulin binding protein. Furthermore, this embodiment shows how a faster purification of the target product can be achieved by combining the virus inactivation step 6 with multiple following downstream process steps 3, here with the depth filter step and the ultrafiltration step 22. In addition, by precipitating DNA and HCP in the virus inactivation step 6 due to the low pH, the life-time of the ultrafiltration membrane 23 can be increased, which results in a more robust and interruption-free process.

If the target product is for example an adeno-associated virus, the centrifugal clarification step 19 and the virus inactivation step 6 may be exchanged by a two-step lysis step.

Another teaching which is of equal importance relates to a system for purifying a target product, particularly a target protein, obtained in a bioprocess 1, from an impurity in a process liquid, in particular by performing the proposed method, wherein the system is designed to purify the target product in several downstream process steps 3, wherein the downstream process steps 3 comprise a virus inactivation step 6, a filtration step 7, a first diafiltration step 8 and a first chromatography step 9, wherein the virus inactivation step 6 comprises an adjustment of at least one parameter of the process liquid, the parameter of the process liquid being suitable for inactivating a virus in the process liquid, wherein the filtration step 7 comprises a filtering of the process liquid through a filter material 11, where at least a part of the impurity is separated from the target product by the filter material 11, wherein the first diafiltration step 8 comprises a filtering of the process liquid through a first diafiltration membrane 12, wherein the first chromatography step 9 comprises a contacting of the process liquid with a first chromatography medium 13, where at least a part of the impurity is separated from the target product by the first chromatography medium 13.

According to one embodiment it is proposed, that the filtration step 7 is performed after the virus inactivation step 6 and the first diafiltration step 8 is performed after the filtration step 7 and the first chromatography step 9 is performed after the first diafiltration step 8, that the pH value of the process liquid reaches a pH value of smaller than or equal to 4.25 in the downstream process steps 3 before the first diafiltration step 8.

The proposed system may be set up to conduct any of the downstream process steps 3 according to the proposed method.

All explanations given with regard to the proposed method are fully applicable.

## Claims

1. Method for purifying a target product, in particular a target protein, obtained in a bioprocess (1), from an impurity in a process liquid, wherein the method comprises purifying the target product in several downstream process steps (3), wherein the downstream process steps (3) comprise a virus inactivation step (6), a filtration step (7), a first diafiltration step (8) and a first chromatography step (9), wherein the virus inactivation step (6) comprises an adjustment of at least one parameter of the process liquid, the parameter of the process liquid being suitable for inactivating a virus in the process liquid,
wherein the filtration step (7) comprises a filtering of the process liquid through a filter material (11), where at least a part of the impurity is separated from the target product by the filter material (11),
wherein the first diafiltration step (8) comprises a filtering of the process liquid through a first diafiltration membrane (12), wherein the first chromatography step (9) comprises a contacting of the process liquid with a first chromatography medium (13), where at least a part of the impurity is separated from the target product by the first chromatography medium (13),
**characterized in**
**that** the filtration step (7) is performed after the virus inactivation step (6) and the first diafiltration step (8) is performed after the filtration step (7) and the first chromatography step (9) is performed after the first diafiltration step (8), that the pH value of the process liquid reaches a pH value of smaller than or equal to 4.25 in the downstream process steps (3) before the first diafiltration step (8).

2. Method according to claim 1, **characterized in that** the target product is an antibody, preferably, that the target protein is a monoclonal antibody.

3. Method according to claim 1 or 2, **characterized in that** all of the downstream process steps (3) are Protein A free.

4. Method according to one of the preceding claims, **characterized in that** the first downstream process step (4) of the downstream process steps (3) is a harvest step (14), preferably, that the harvest step (14) involves the harvest of mammalian cells, in particular CHO cells, and, that the last downstream process step (5) of the downstream process steps (3) is a final product collection step (15).

5. Method according to one of the preceding claims, **characterized in that** the at least one parameter of the process liquid in the virus inactivation step (6) is the temperature of the process liquid and/or the concentration of a virus inactivating agent in the process liquid, preferably, that the virus inactivating agent is a pH altering substance and/or a solvent and/or a detergent.

6. Method according to one of the preceding claims, **characterized in that** the pH value of the process liquid remains constant between the virus inactivation step (6) and the first diafiltration step (8), preferably, that no pH altering substance is added to the process liquid between the virus inactivation step (6) and the first diafiltration step (8).

7. Method according to one of the claims 5 or 6, **characterized in that** the pH value of the process liquid is adjusted in the virus inactivation step (6) to an inactivation pH value of smaller than or equal to 4.25, in particular to a pH value of above 3.1 and/or below 3.6, more preferably, 3.4, by addition of the pH altering substance to the process liquid.

8. Method according to one of the preceding claims, **characterized in that** the first diafiltration step (8) involves an adjustment of the pH value of the process liquid, preferably, that the pH value of the process liquid is adjusted in the first diafiltration step (8) to a pH value which is higher than the inactivation pH value of the virus inactivation step (6), more preferably, that the pH value of the process liquid is adjusted in the first diafiltration step (8) to a pH value of higher or equal to 4.25, preferably higher or equal to 4.5, more preferably higher or equal to 5.0, most preferably higher or equal to 5.5
and/or,
that the first diafiltration step (8) involves an adjustment of the conductivity value of the process liquid, preferably, that the conductivity value of the process liquid is adjusted in the first diafiltration step (8) to a value of lower or equal to 25.0 mS/cm, preferably lower or equal to 20.0 mS/cm, more preferably lower or equal to 15.0 mS/cm, more preferably lower or equal to 10.0 mS/cm, most preferably lower or equal to 5.0 mS/cm.

9. Method according to one of the preceding claims, **characterized in that** the first chromatography step (9) is the first chromatography step (9) of the downstream process steps (3), and/or, that the first chromatography step (9) is directly followed by a group of interrelated further chromatography steps (16), wherein the group of interrelated further chromatography steps (16) comprises at least a second chromatography step (17), wherein the second chromatography step (17) comprises a contacting of the process liquid with a second chromatography medium (18), where at least a part of the impurity is separated from the target product by the second chromatography medium (18), more preferably, that the last chromatography step of the group of interrelated further chromatography steps (16) is the last chromatography step of the downstream process steps (3).

10. Method according to one of the preceding claims, **characterized in that** the first chromatography medium (13) and/or the second chromatography medium (18) is a cation exchange chromatography medium, an anion exchange chromatography medium, a hydrophobic chromatography medium, a mixed-mode chromatography medium, or an affinity chromatography medium, preferably, that the first chromatography medium (13) is a cation exchange chromatography medium, and, that the second chromatography medium (18) is an anion exchange chromatography medium, more preferably, that the first chromatography medium (13) and the second chromatography medium (18) are immunoglobulin binding protein free, in particular Protein A free, more preferably, that any one of the chromatography steps of the downstream process steps (3) is immunoglobulin binding protein free, in particular Protein A free.

11. Method according to one of the preceding claims, **characterized in that** the downstream process steps (3) further comprise a centrifugal clarification step (19), where at least a part of the impurity is separated from the target product by a centrifuge (20), preferably, that the centrifuge (20) is a continuous centrifuge (21), in particular a fluidized bed centrifuge, more preferably, that the centrifugal clarification step (19) is performed before the virus inactivation step (6).

12. Method according to one of the preceding claims, **characterized in that** the downstream process steps (3) further comprise an ultrafiltration step (22), wherein the ultrafiltration step (22) comprises a filtering of the process liquid through an ultrafiltration membrane (23), wherein the ultrafiltration step (22) comprises an increase in the concentration of the target product in the process liquid, preferably, that the ultrafiltration step (22) is performed before the first diafiltration step (8), more preferably, that the ultrafiltration step (22) is performed between the filtration step (7) and the first diafiltration step (8).

13. Method according to one of the preceding claims, **characterized in that** the downstream process steps (3) further comprise a flocculation step, wherein the flocculation step comprises an addition of a flocculation inducing agent to the process liquid, where at least a part of the impurity is flocculated by the flocculation inducing agent, preferably, that the flocculation step and the virus inactivation step (6) are a combined step, or, that the flocculation step is performed directly after the virus inactivation step (6).

14. Method according to one of the preceding claims, **characterized in that** the ultrafiltration step (22) and the first diafiltration step (8) are performed in a single-pass-mode, in which the process liquid passes the ultrafiltration membrane (23) and the first diafiltration membrane (12) only once, that the ultrafiltration step (22) is followed by the first diafiltration step (8), in particular directly followed by the first diafiltration step (8), or, that the first diafiltration step (8) is followed by the ultrafiltration step (22), in particular directly followed by the ultrafiltration step (22), preferably, that the ultrafiltration membrane (23) and the first diafiltration membrane (12) are continuously flown through by the process liquid, more preferably, that the ultrafiltration membrane (23) and the first diafiltration membrane (12) are housed in one module.

15. System for purifying a target product, particularly a target protein, obtained in a bioprocess (1), from an impurity in a process liquid, in particular by performing the method according to one of the preceding claims, wherein the system is designed to purify the target product in several downstream process steps (3), wherein the downstream process steps (3) comprise a virus inactivation step (6), a filtration step (7), a first diafiltration step (8) and a first chromatography step (9), wherein the virus inactivation step (6) comprises an adjustment of at least one parameter of the process liquid, the parameter of the process liquid being suitable for inactivating a virus in the process liquid, wherein the filtration step (7) comprises a filtering of the process liquid through a filter material (11), where at least a part of the impurity is separated from the target product by the filter material (11), wherein the first diafiltration step (8) comprises a filtering of the process liquid through a first diafiltration membrane (12),
wherein the first chromatography step (9) comprises a contacting of the process liquid with a first chromatography medium (13), where at least a part of the impurity is separated from the target product by the first chromatography medium (13),
**characterized in**
**that** the filtration step (7) is performed after the virus inactivation step (6) and the first diafiltration step (8) is performed after the filtration step (7) and the first chromatography step (9) is performed after the first diafiltration step (8), that the pH value of the process liquid reaches a pH value of smaller than or equal to 4.25 in the downstream process steps (3) before the first diafiltration step (8).
